# EUROPEAN PATENT SPECIFICATION

(11) **EP 0 413 414 B1**
(45) Date of publication and mention of the grant of the patent: **20.04.1994**
(21) Application number: 90306191.9
(22) Date of filing: 07.06.1990
(51) Int. Cl.: A61M 5/50

(54) **Safety hypodermic syringe**
Sicherheitsinjektionsspritze
Seringue à injections de haute sécurité

(30) Priority: 14.08.1989 US 393390
(43) Date of publication of application: 20.02.1991
(73) Proprietor: Tsao, Chien-Hua, Taipei (TW)
(72) Inventor: Tsao, Chien-Hua, Taipei (TW)
(74) Representative: Thomson, Roger Bruce

(56) References cited:
- EP-A- 0 307 367
- WO-A-89/00435
- CH-A- 669 910
- GB-A- 2 197 792
- US-A- 4 804 370
- US-A- 4 838 869

## Description

The present invention relates to hypodermic syringes, and more particularly to a safety hypodermic syringe in which the hypodermic needle is automatically concealed therein after the injection process has been performed.

The hypodermic syringes currently in use are normally disposable and made of plastics material. They are intended for single-shot use and to be thrown away immediately after use in order to prevent contact with body fluids.

The conventional plastics syringe changes little in structure as between before and after injection. Therefore, mistakes tend to happen and a used syringe may be repeatedly used, or, in order to save cost, unscrupulous people may wash used syringes for further use. As is well known, it is quite dangerous repeatedly to use a hypodermic syringe since infections such as AIDS and hepatitis may be passed through contact with body fluids.

Another disadvantage of the conventional disposable hypodermic syringe is the high frequency of accidents to doctors and nurses due to them pricking themselves with the needle when using the syringe. According to the prior art, the hypodermic needle is normally protected by a needle cap which has to be removed from the hypodermic needle before sucking up liquid medicine. After the injection, the needle cap should be re-fitted on the hypodermic needle before the syringe is thrown away, in order to prevent pricking accidents to anyone who handles the refuse. However, because the bore of a needle cap is very small, the hypodermic needle is difficult to insert. Therefore, pricking accidents may happen during the re-fitting of a needle cap on to a hypodermic needle. Such accidents will occur more frequently when a nurse is working under emergency conditions or high pressure. According to the report from "Morbidity and Mortality Weekly Report" (Vol. 36, No. 2S) issued on August 21, 1987 by the Department of Health of the U.S. Government, it was disclosed that 708 persons had been injured by hypodermic needles among the total of 883 investigated, i.e. 80% of those investigated had at some time been pricked by a hypodermic needle. Furthermore, according to the report from "The New England Journal of Medicine" (Vol. 31 ), it is a high risk matter to be injured by a used hypodermic needle since infections such as AIDS and hepatitis may be passed through contact with body fluids.

According to the statistics on AIDS cases issued by the Department of Health of the U.S. Government, up to May 1988 there were 68,052 AIDS patients reported, of which 34,088 had died. It is estimated that there will be as many as one and half million AIDS carrier patients in 1991. Therefore, it is very important to protect medical treatment and nursing staff and people handling medical refuse against contact with body fluids.

US-A-4838869 describes a retractable hypodermic needle for one-time use wherein the needle is spring-loaded and automatically retracted into the syringe when the plunger is fully depressed. Protrusions on the end of the plunger engage tabs holding the spring-loaded needle to release the needle for retraction.

WO89/00435 describes a disposable hypodermic syringe in which the liquid chamber has a yieldable end wall and the end of the needle opposite its point has a head which seals a hole in the end wall. When the plunger strikes the end wall it interlocks with it and displaces the end wall so that the needle travels under spring force into a closed cavity in the plunger.

The main object of the present invention is to provide a safety hypodermic syringe in which the hypodermic needle will, after use, automatically drop into the inner chamber of the plunger to isolate it from contact with people, so that it can then easily be thrown away, and so as to prevent body fluid contact.

Another object of the present invention is to provide a safety hypodermic syringe which will change in external appearance upon use, with the hypodermic needle dropping completely into the syringe when it is used, so as to help nursing staff as well as patients easily identify if a syringe is a new one or has been used.

In accordance with the invention there is provided a safety hypodermic syringe comprising:
a barrel for containing liquid having an inner surface, an outer surface, a rear opening and a front neck portion of reduced diameter;
clamping means fixedly set in said neck portion comprising resilient clamping means constantly exerting a clamping force inwards;
a holder connected to a hypodermic needle, said holder being firmly retained by said clamping means to allow said needle to protrude beyond said barrel in a position ready for injection;
bias means exerting an axial displacement force on said holder in a rearward direction,
and a hollow plunger having at least a part thereof inserted through said rear opening and received in said barrel to slide therein, said hollow plunger comprising an inner chamber and a front end surface having a hole therethrough communicating with said inner chamber, said hole
normally being blocked by a cover;
wherein, when said plunger is pushed towards said front neck portion of said barrel, the front end surface of said plunger presses against said clamping means to open the clamping means and release the holder from the constraint of said clamping means, enabling said bias means to force said hypodermic needle into the inner chamber of the plunger, characterised in that the cover comprises a displaceable plug and the holder is provided with protruding means extending axially towards the plunger, whereby substantially simultaneously with the plunger opening the clamping means the protruding means completely remove the plug from the hole to provide clear access to the inner chamber for the needle.

In order that the invention may be fully understood, a preferred embodiment of syringe will now be described by way of example and with reference to the accompanying drawings, in which:
Fig. 1 is a perspective exploded and partly sectional view of a safety hypodermic syringe in accordance with the present invention;
Fig. 2 is a longitudinal sectional view of the safety hypodermic syringe before the injection;
Fig. 3 is a longitudinal sectional view of the safety hypodermic syringe when about to finish the injection; and,
Fig. 4 is a longitudinal sectional view of the safety hypodermic syringe after the injection with the hypodermic needle having dropped into the hollow plunger assembly.

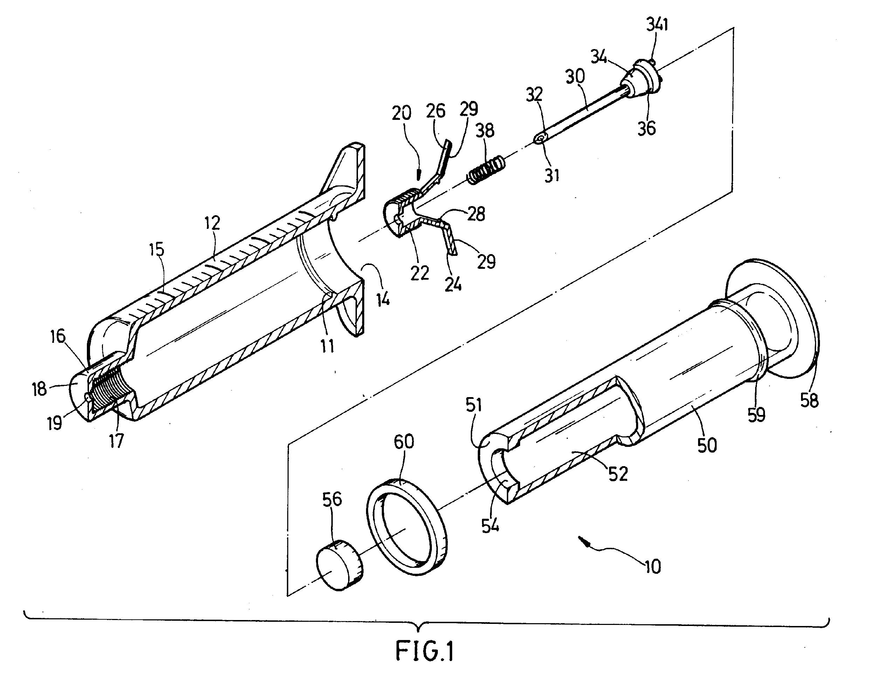

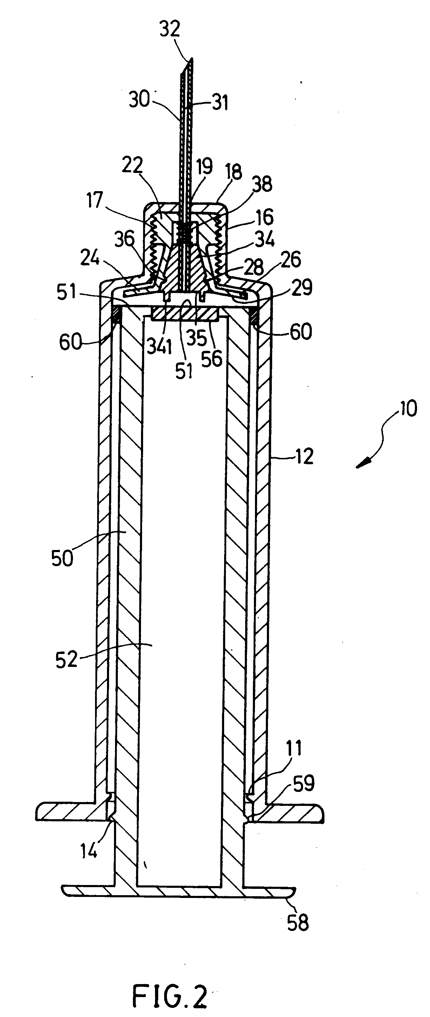

Referring to Figs. 1 and 2, a safety hypodermic syringe 10 comprises a barrel 12 for containing liquid medicine. The barrel 12 has an inner surface, an outer surface, an opening 14 at the rear end and a neck portion 16 of reduced diameter at the front end. The neck portion 16 has an inner thread 17 and has an axial hole 19 through which a hypodermic needle 30 extends. An elastic clamping means 20 is connected to the inside of the barrel 12 by means of a screw joint, having integrally a cylindrical front portion with an outer thread 22 for connection with the inner thread 17 and a rear portion composed of two L-shaped clamping elements 24 and 26. The elastic clamping means 20 provides elastic resilience to clamping inwards. The clamping means 20 may be incorporated with the barrel 12 to form a unitary part. The hypodermic needle 30 which is fixedly connected to a holder 34 firmly retained by the two suspended and elastic clamping elements 24 and 26 has an internal passage through which liquid medicine passes, and a sharp front end 32 which serves as a means to insert the needle into a patient's muscle for the injection of liquid medicine from the barrel 12 through the passage 31 into the patient's body. In order to reinforce the clamping effect of the clamping elements 24 and 26 on the holder 34, the clamping elements 24 and 26 have flanges 28 thereon to engage respectively with recesses 36 which are provided in the holder 34. Therefore, under normal conditions, when the two clamping elements are not prised open by external forces, the holder 34 will be firmly retained by the elastic clamping means 20 by means of the engagement of the flanges 28 with the recesses 36 and the constant inward clamping force of the clamping means 20 itself, so as to let the hypodermic needle 30 protrude beyond the axial hole 19 of the barrel 12 and in a position ready for the injection. The holder 34 includes at least one protrusion 341 at the bottom surface 35 thereof. An elastic element 38, like a spring, is mounted on the hypodermic needle 30 and is seated between the holder 34 and the externally threaded cylindrical front portion 22. The elastic element 38 exerts an axial bias force on the holder 34. Under normal conditions, the elastic force that the elastic element 38 provides is overcome by the clamping force that the two clamping means 24 and 26 provide. Therefore, the elastic force will not be dominant before the injection process is performed. The safety syringe 10 further comprises a hollow plunger 50 having an annular plunger element 60 made integral therewith at the front end by means of heat-sealing or any other suitable process. The plunger assembly which is formed by the hollow plunger 50 and the plunger element 60 may be set into the barrel 12 from the opening 14 by means of a push force to slide it axially therein, with the front end surface 51 of the hollow plunger 50 and the surrounding plunger element 60 together filling the opening 14 of the barrel 12. Therefore, by axial movement of the hollow plunger 50 and the plunger element 60, liquid medicine may be sucked up into the barrel 12 or liquid medicine in the barrel 12 may be injected through the passage 31 of the hypodermic needle 30. According to the present invention, the hollow plunger 30 has an inner chamber 52, a front end opening 54 in the front end surface 51 to communicate with the inner chamber 52, which front end opening 54 is normally sealed by a covering means 56, and an enclosed hand-grip portion 58.

The hypodermic needle 30 which protrudes beyond the barrel 12 may be enclosed by a needle cap (not shown) to protect against pricking accidents and prevent contamination. Even though pricking accidents may occur, contamination is unlikely to happen.

Referring to Fig. 2, before the injection process has been performed, the needle-connected holder 34 is firmly retained by the clamping means 20 in a ready position. For use, the plunger 50 is pulled backwards to produce a vacuum suction so as to suck up prepared liquid medicine into the barrel 12 through the needle passage 31.

To administer the injection, the pointed front end 32 of the hypodermic needle 30 is pushed into the patient's body. When the hypodermic needle 30 pierces the patient's skin, the reaction force on the hypodermic needle 30 will be absorbed by the clamping force that the clamping means 20 applies to the holder 34. As soon as the plunger 50 is pushed forwards, the liquid medicine in the barrel 12 is forced through the passage 31 into the patient's body.

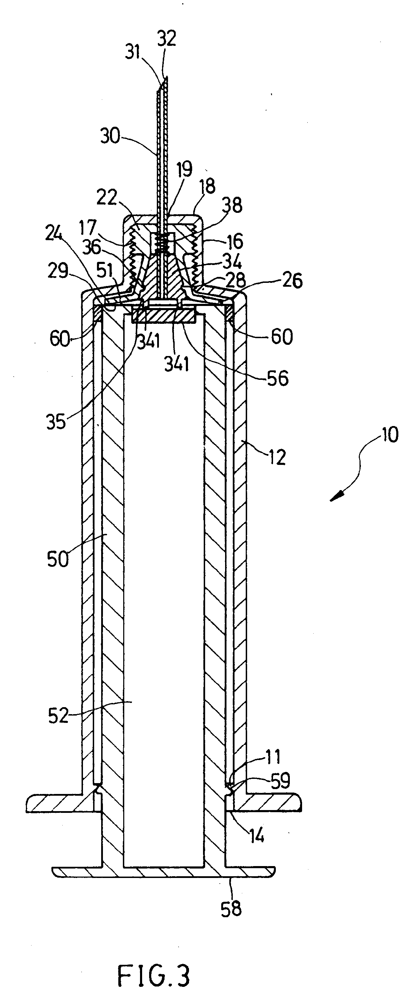

With reference to Fig.3, when the plunger 50 is pushed towards its forward limit, before the bottom surface 29 of the two clamping elements 24 and 26 comes into contact with the plunger 50, the protrusions 341 on the bottom surface 35 of the holder 34 will first come into contact with the covering means 56 which blocks the front end opening 54 of the plunger 50. Therefore, as soon as the plunger 50 is pushed to its forward limit, the covering means 56 is pushed to break away from the front end opening 54, or is punched through.

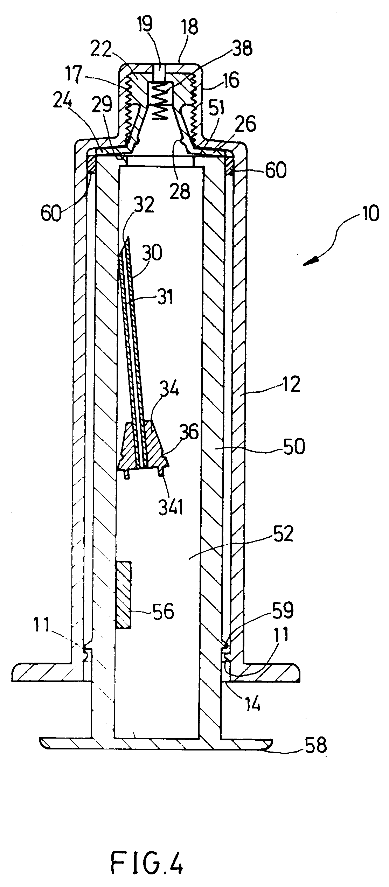

With reference to Fig. 4, when the plunger 50 is pushed to its forward limit, the covering means 56 is squeezed out of or punched through the front end opening 54, and the front end surface 51 of the plunger 50 presses the bottom surface 29 of the two L-shaped clamping elements 24 and 26 to prise them apart and to let the flanges 28 break away from engagement with the recesses 36, so as to let the holder 34 be released from the constraint of the clamping means 20. Under these conditions, only the muscle of the patient (not shown) provides a retaining force to hold the hypodermic needle 30 and let the hypodermic needle 30 and the connected holder 34 be temporarily retained in the injection position. After the injection process has been completed, the hypodermic needle 30 is pulled out of the patient's body and the spring force from the elastic element 38 will immediately push the holder 34 to cause the hypodermic needle 30 to move axially rearwards. Because the covering means 56 has been punched through the front end opening 54, the hypodermic needle 30 will be pushed by the elastic element 38 into the inner chamber 52 of the plunger 50 to become totally received therein.

The barrel 12 and the plunger 50 have wedging rings 11 and 59 matching each other to prevent a return stroke of the plunger 50 when the plunger 50 has been pushed to its forward limit, so as to prevent the plunger 50 from breaking away from the barrel 12 and to reduce the overall volume of the used syringe.

The barrel 12 has a volumetric scale 15 on the outer wall surface for identification of the amount of liquid medicine drawn into the barrel.

As shown in Figs. 2 to 4, when a nurse pulls the hypodermic needle 30 and the syringe 10 out of a patient's body to complete the injection process, the holder 34 of the hypodermic needle 30 becomes freely movable because it no longer has any clamping force applied thereto and is immediately pushed by the elastic element 38 into the inner chamber 52 of the plunger 50 to conceal it therein. Therefore, after having completed an injection, the used syringe isolates the hypodermic needle from contact with people so as to prevent pricking accidents or body fluid contact.

## Claims

1. A safety hypodermic syringe comprising:
a barrel (12) for containing liquid having an inner surface, an outer surface, a rear opening (14) and a front neck portion (16) of reduced diameter;
clamping means (20) fixedly set in said neck portion comprising resilient clamping means (24,26) constantly exerting a clamping force inwards;
a holder (34) connected to a hypodermic needle (30), said holder being firmly retained by said clamping means (24, 26) to allow said needle to protrude beyond said barrel (12) in a position ready for injection;
bias means (38) exerting an axial displacement force on said holder in a rearward direction,
and a hollow plunger (50) having at least a part thereof inserted through said rear opening (14) and received in said barrel (12) to slide therein, said hollow plunger comprising an inner chamber (52) and a front end surface (51) having a hole (54) therethrough communicating with said inner chamber, said hole normally being blocked by a cover (56);
wherein, when said plunger (50) is pushed towards said front neck portion (16) of said barrel, the front end surface (51 ) of said plunger presses against said clamping means (24, 26) to open the clamping means and release the holder from the constraint of said clamping means, enabling said bias means (38) to force said hypodermic needle (30) into the inner chamber (52) of the plunger, characterised in that the cover comprises a displaceable plug (56) and the holder (34) is provided with protruding means (341) extending axially towards the plunger, whereby substantially simultaneously with the plunger opening the clamping means the protruding means (341) completely remove the plug (56) from the hole (54) to provide clear access to the inner chamber (52) for the needle.

2. A safety hypodermic syringe according to claim 1, characterised in that said barrel (12) and said plunger (50) have check means (11, 59) to prevent a return stroke of the plunger when said plunger is pushed into contact with said holder (34).

3. A safety hypodermic syringe according to claim 2, characterised in that said check means includes two engaging wedging rings (11, 59) respectively on the inner surface of said barrel (12) and on the outer surface of said plunger (50).

4. A safety hypodermic syringe according to claim 1, 2 or 3, characterised in that said clamping means includes retainer flanges (28) and said holder has recesses (36) therein to engage with said retainer flanges when said holder is retained by said clamping means.

5. A safety hypodermic syringe according to any preceding claim, characterised in that said clamping means (20) is incorporated with said barrel (12) to form a one-piece element.

## Patentansprüche

1. Sicherheitsinjektionsspritze, die die nachgenannten Bestandteile umfaßt:
einen Zylinder (12) zur Flüssigkeitsaufnahme mit einer inneren Oberfläche, einer äußeren Oberfläche, einer rückwärtigen Öffnung (14) und einem vorderen Halsteil (16) von reduziertem Durchmesser;
Klemmittel (20), die in dem erwähnten Halsteil fest angeordnet sind und elastische Klemmschenkel (24,26) aufweisen, die eine nach innen gerichtete Klemmkraft hervorrufen;
einen mit einer Injektionsnadel (30) verbundenen Halter (34), der durch die Klemmschenkel (24,26) so fest gehalten wird, daß die Nadel aus dem Zylinder (12) in einer für die Injektion bereiten Position herausragt;
Federmittel (38), die eine axiale Verschiebekraft auf den erwähnten Halter in rückwärtiger Richtung ausüben, und einen hohlen Kolben (50), der zumindest teilweise durch die rückwärtige Öffnung (14) in den Zylinder (12) einzusetzen und darin gleitverschieblich ist, wobei der erwähnte Hohlkolben eine Innenkammer (52) und ein vorderes Stirnende (51) aufweist, das eine mit der Innenkammer in Verbindung stehende Öffnung (54) aufweist, die normalerweise durch einen Deckel (56) verschlossen ist;
wobei der Kolben (50), wenn er in Richtung zu dem erwähnten Halsteil (16) des Zylinders gestoßen wird, mit seiner vorderen Stirnfläche (51) gegen die Klemmschenkel (24,26) stößt, um das Klemmittel zu öffnen und den Halter von der Einzwängung durch die Klemmittel zu befreien und dadurch die Federmittel (38) zu veranlassen, die Injektionsnadel (30) in die Innenkammer (52) des Kolbens zu zwingen, **da****durch gekennzeichnet,** daß der Deckel aus einem lageverdrängbaren Stopfen (56) besteht und der Halter (34) mit Vorsprüngen (341) versehen ist, die sich zum Kolben hin axial erstrecken, wobei im wesentlichen gleichzeitig mit dem durch den Kolben bewirkten Öffnen der Klemmittel die Vorsprüngen (341) den Stopfen (56) vollständig aus der Öffnung (54) entfernen, um der Injektionsnadel einen freien Zugang zur Innenkammer (52) zu verschaffen.

2. Sicherheitsinjektionsspritze nach Anspruch 1, **dadurch ge****kennzeichnet,** daß der Zylinder (12) und der Kolben (50) Bremsmittel (11,59) aufweisen, um einen Rückwärtsstoß des Kolbens zu verhindern, wenn dieser gegen den Halter (34) gedrückt wird.

3. Sicherheitsinjektionsspritze nach Anspruch 2, **dadurch ge****kennzeichnet,** daß die Haltemittel aus zwei zusammenwirkenden Klemmringen (11,59) bestehen, die auf der Innenoberfläche des Zylinders (12) und der Außenoberfläche des Kolbens (50) vorhanden sind.

4. Sicherheitsinjektionsspritze nach Anspruch 1,2 oder 3, **da****durch gekennzeichnet,** daß die erwähnten Klemmschenkel Rückhaltevorsprünge (28) und der erwähnte Halter eine damit in Eingriff zu bringende Rille (36) aufweisen, solange der Halter von den Klemmschenkeln gehalten wird.

5. Sicherheitsinjektionsspritze nach einem der vorerwähnten Ansprüche, **dadurch gekennzeichnet,** daß die Klemmittel (20) mit dem Zylinder (12) zu einer einstückigen Baueinheit verbunden sind.

## Revendications

1. Seringue hypodermique de sûreté comportant :
un corps (12) destiné à contenir un liquide et ayant une surface intérieure, une surface extérieure, une ouverture arrière (14) et une partie de col avant (16) de diamètre réduit ;
des moyens de serrage (20) montés fixement dans ladite partie de col et comportant des moyens élastiques (24, 26) de serrage exerçant constamment une force de serrage vers l'intérieur ;
une monture (34) reliée à une aiguille hypodermique (30), ladite monture étant retenue fermement par lesdits moyens de serrage (24, 26) pour permettre à ladite aiguille de dépasser au-delà dudit corps (12) dans une position prête pour une injection ;
un moyen de rappel (38) exerçant une force de déplacement axial sur ladite monture vers l'arrière, et
un plongeur creux (50) dont au moins une partie est insérée à travers ladite ouverture arrière (14) et est reçue dans ledit corps (12) pour y coulisser, ledit plongeur creux comprenant une chambre intérieure (52) et une surface extrême avant (51) traversée par un trou (54) communiquant avec ladite chambre intérieure, ledit trou étant normalement fermé par un capuchon (56) ;
dans laquelle, lorsque ledit plongeur (50) est poussé vers ladite partie de col avant (16) dudit corps, la surface extrême avant (51) dudit plongeur exerce une pression contre lesdits moyens de serrage (24, 26) pour ouvrir les moyens de serrage et libérer la monture de la contrainte desdits moyens de serrage, permettant audit moyen de rappel (38) d'introduire à force ladite aiguille hypodermique (30) dans la chambre intérieure (52) du plongeur, caractérisée en ce que le capuchon comporte un obturateur (56) pouvant être déplacé et la monture (34) est pourvue de moyens en saillie (341) s'étendant axialement vers le plongeur, afin que, pratiquement en même temps que le plongeur ouvre les moyens de serrage, les moyens en saillie (341) enlèvent complètement l'obturateur (56) du trou (54) pour permettre un accès dégagé à la chambre intérieure (52) pour l'aiguille.

2. Seringue hypodermique de sûreté selon la revendication 1, caractérisée en ce que ledit corps (12) et ledit plongeur (50) comportent des moyens de retenue (11, 59) destinés à empêcher une course de retour du plongeur lorsque ledit plongeur est poussé jusqu'en contact avec ladite monture (34).

3. Seringue hypodermique de sûreté selon la revendication 2, caractérisée en ce que lesdits moyens de retenue comprennent deux anneaux (11, 59) s'enclenchant par effet de coin, situés respectivement sur la surface intérieure dudit corps (12) et sur la surface extérieure dudit plongeur (50).

4. Seringue hypodermique de sûreté selon la revendication 1, 2 ou 3, caractérisée en ce que lesdits moyens de serrage comprennent des rebords (28) de retenue et ladite monture présente des évidements (36) destinés à s'enclencher avec lesdits rebords de retenue lorsque ladite monture est retenue par lesdits moyens de serrage.

5. Seringue hypodermique de sûreté selon l'une quelconque des revendications précédentes, caractérisée en ce que lesdits moyens de serrage (20) sont incorporés avec ledit corps (12) pour former un élément d'une seule pièce.
